# EUROPEAN PATENT APPLICATION

(11) **EP 3 210 620 A1**
(43) Date of publication of application: **30.08.2017**
(21) Application number: 17161071.0
(22) Date of filing: 03.08.2005
(51) Int. Cl.: A61K 38/48, A61K 8/99, A61K 9/00, A61K 47/26, A61Q 7/00, A61Q 19/08, A61P 1/00, A61P 5/00, A61P 11/00, A61P 13/00, A61P 17/00, A61P 21/00, A61P 25/14, A61P 27/00, A61P 29/00, A61P 37/00

(54) **PHARMACEUTICAL COMPOSITION CONTAINING BOTULINUM NEUROTOXIN A2**

(30) Priority: 04.08.2004 GB 0417367; 24.09.2004 GB 0421290; 24.09.2004 GB 0421288; 28.10.2004 GB 0423952; 28.10.2004 GB 0423953
(62) Divisional of application: 14180875.8
(71) Applicant: Ipsen Biopharm Limited, Wrexham LL13 9UF (GB)
(72) Inventor: PANJWANI, Naveed, CHESTER, CH1 2BQ (GB); WEBB, Paul, deceased (GB); PICKETT, Andrew, WREXHAM, LL12 8DU (GB)
(74) Representative: Grot, Emmanuelle

(57) **Abstract**

A composition, medicament or use, comprising or consisting of botulinum toxin type A2 and a surfactant, having one or more or all of the characteristics selected from the group consisting of :
(a) a much longer duration of action;
(b) a much faster rate of onset of muscular paralysis;
(c) a significantly greater intramuscular safety margin;
(d) a selective action on inhibition of skeletal muscle contraction;
(e) a selective action on inhibition of pain-related (nociceptive) nerve cell function, wherein each characteristic is determined comparative to the same characteristic effect of botulinum toxin type A1.

## Description

The invention relates to a pharmaceutical composition containing botulinum neurotoxin.

The presently most used botulinum neurotoxin is botulinum neurotoxin type A. This neurotoxin is produced during fermentation in the presence of *Clostridium botulinum* strains. Botulinum neurotoxin type A complexes (which include botulinum neurotoxin type A and at least another non-toxic protein) are active principles widely used in modem medicine. An example of a pharmaceutical composition based on such a complex is the product Dysport^{®} currently sold by the company of the Applicants. Among the most common medical indications for which a botulinum neurotoxin type A complex could be used, one could mention the treatment of a number of muscle disorders (e.g. blepharospasm, hemifacial spasm, torticollis, spasticity, tension headache, back pain or wrinkles), as well as other disorders such as migraine. Alternatively, high purity botulinum toxin (i.e. botulinum neurotoxin free from its complexing non-toxic proteins) may replace the corresponding botulinum toxin complex as disclosed in PCT applications WO 96/11699 or WO 97/35604.

Currently, the marketed botulinum neurotoxin compositions contain human serum albumin. However, some concerns have been expressed about albumin (see e.g. in PCT application WO 01/58472). For this reason, the pharmaceutical industry is now considering to find alternative stabilising agents to albumin by other stabilising agents in pharmaceutical compositions.

A possible solution is disclosed in PCT patent application WO 01/58472. In this document, albumin is replaced by a polysaccharide, i.e. a polymer of more than two saccharide molecule monomers, which plays the role of the stabiliser in the botulinum neurotoxin composition.

An alternative solution is the one described in PCT patent application WO 97/35604 or US patents Nos. 5,512,547 and 5,756,468. In these documents, it is disclosed that pure botulinum neurotoxin (i.e. botulinum neurotoxin free from its complexing non-toxic proteins) can be stabilised by trehalose.

The Applicant has unexpectedly discovered that a surfactant possesses sufficient stabilising effects to replace albumin, the polysaccharide of PCT patent application WO 01/58472 or the trehalose of PCT patent application WO 97/35604 in botulinum neurotoxin compositions.

The invention therefore pertains to the use of a surfactant for stabilising a solid or liquid pharmaceutical composition that contains as active principle a botulinum toxin.

By botulinum toxin should be understood a naturally occurring botulinum toxin or any recombinantly produced botulinum toxin.

By naturally occurring botulinum toxin should be understood either a high purity botulinum neurotoxin derived from *Clostridium spp* or a botulinum neurotoxin complex derived from *Clostridium spp.*

By high purity botulinum neurotoxin is meant, in the present application, botulinum neurotoxin outside from complexes including at least another protein. In other words, a high purity botulinum neurotoxin does not contain significant quantities of any other *Clostridium spp* derived protein than botulinum neurotoxin.

Further, according to the present invention, botulinum neurotoxin complexes and high purity botulinum neurotoxins will be botulinum neurotoxin complex and high purity botulinum neurotoxin of type A2.

The classical type A botulinum toxin (i.e. the active principle of the marketed products Dysport and Botox) is increasingly being referred to by those skilled in the art as type A1 botulinum toxin. This is to distinguish it from type A2 botulinum toxin originally isolated from infant botulism cases in 1990, which is an immunologically and biochemically distinct botulinum toxin. In the instant patent application, we will therefore be using this nomenclature.

Clostridium botulinum type A2 toxin-producing organisms were first identified in 1990 in Japan, from multiple cases of infant botulism (Sakaguchi et al., Int. J. Food Microbiol. (1990), 11, 231-242). Infant botulism, or intestinal colonisation botulism is unlike food-borne botulism in that the toxin is produced after infection of the patient, rather than pre-formed in food. The clinical isolate strains most closely associated with type A2 toxin are Kyoto-F, Chiba-H, Y-8036, 7103-H, 7105-H and KZ1828, although several others have been characterized as type A2 by molecular methods (Cordoba et al., System. Appl. Microbiol. (1995), 18, 13-22; Franciosa et al., abstract presented at 40th Interagency Botulism Research Coordinating Committee (IBRCC) Meeting, November 2003).

Botulinum type A2 toxin is a unique neurotoxin which has been shown to be a distinct toxin type when compared with other botulinum toxin types A - G. Botulinum type A2 toxin differs from type A1 toxin in its molecular genetic characteristics, its biochemical characteristics and in its immunological characteristics.

At the molecular genetic level, the organisation of the type A2 neurotoxin gene cluster is distinct from all other botulinum toxin types. Many botulinum toxin types, including type A botulinum toxins, are found as neurotoxin complexes with haemagglutinin (HA) proteins as components of the complex. The genes encoding these HA proteins (HA17, HA34 and HA70) are contained in the neurotoxin gene cluster of type A, B, C, D and G organisms, but are entirely absent in the type A2 neurotoxin gene cluster. The type A2 neurotoxin gene cluster also contains regulatory genes such as p47, which are absent in type A1 neurotoxin gene clusters. Additionally, the sequence of the NTNH protein of type A2 toxin complex has been shown to be a mosaic of type C and type A1 NTNH gene sequences (Kubota et al., Biochem. Biophys. Res. Commun. (1996), 224(3), 843-848).

Type A2 toxin and type A1 toxin also differ markedly in the biochemical characteristics of the purified toxin complex. While type A1 toxin complex contains the NTNH protein, and at least three HA proteins (HA17, HA34 and HA70), type A2 toxin complex contains only an NTNH protein and lacks the HA proteins (Sakaguchi et al., Int. J. Food Microbiol. (1990), 11, 231-242). The neurotoxin molecule itself differs in molecular weight, the heavy chain being 101 kDa in type A2 toxin and 93 kDa in type A1 toxin , and shows differing sensitivity to proteases (Kozaki et al., Microbiol. Immunol. (1995), 39(10), 767-74). The amino acid sequence of the type A2 and type A1 toxins are markedly different, particularly in the heavy chain region, where 109 of the 847 amino acids are different between the two toxin types (13% difference) (Cordoba et al., System. Appl. Microbiol. (1995), 18, 13-22). The heavy chain sequences of isolates of type A1 toxins, by contrast, typically differ by less than 2%. Heavy chain of botulinum neurotoxins are responsible for key biological functions of the molecule, including receptor binding on target cells and intracellular trafficking (Zhang et al., Gene (2003), 315, 21-32). Indeed, studies of binding of neurotoxins A2 and A1 have shown different binding characteristics of the two toxins to purified synaptosomes (Kozaki et al., Microbiol. Immunol. (1995), 39(10), 767-74).

Botulinum type A2 toxin is also immunologically distinct. Antibodies raised against type A toxin have been shown not to recognise type A2 botulinum toxin (and vice versa) in immunodiffusion experiments, ELISA and Western blots (Sakaguchi et al., Int. J. Food Microbiol. (1990), 11, 231-242; Kozaki et al., Microbiol. Immunol. (1995), 39(10), 767-74). Most significantly, however, antibodies raised to type A1 toxins, while able to neutralize toxicity of type A1 toxin in mice, could not neutralize type A2 toxins in parallel mouse toxicity experiments (Kozaki et al., Microbiol. Immunol. (1995), 39(10), 767-74).

In summary, it can be seen from the state of the art that type A2 botulinum toxin is biochemically and immunologically different from other botulinum toxin types, and particularly from type A1 botulinum toxin.

The high purity botulinum neurotoxin type A2 used according to the invention or contained in pharmaceutical compositions can easily be obtained from the corresponding botulinum neurotoxin complex, for example as explained in Current topics in Microbiology and Immunology (1995), 195, p. 151-154. High purity *Clostridium botulinum* toxin is obtained, for example, by purification of an adequate fermentation medium (for example, an enriched meat media broth containing *Clostridium Botulinum* and left for fermentation - this broth may be, for example, the one described in Current topics in Microbiology and Immunology (1995), 195, p. 150 and DasGupta, "Microbial food toxicants. Clostridium botulinum toxins. CRC handbook of foodborne diseases of biological origin", CRC Boca Raton, p. 25-56). When including high purity botulinum neurotoxin in a composition according to the instant invention, the purity degree of the toxin should preferably be higher than 80%, more preferably higher than 90 or 95% and in a more particularly preferred manner higher than 98% or 99%. It can be assessed, for example, by using the purity assay described in the present application.

The instant invention relates to a solid or liquid pharmaceutical composition comprising:
(a) a botulinum toxin type A2, and
(b) a surfactant.

According to a particular variant of the invention, the pharmaceutical composition will be a solid pharmaceutical composition and will essentially consist in:
(a) a botulinum toxin type A2, and
(b) a surfactant.

According to another particular variant of the invention, the pharmaceutical composition will be a liquid pharmaceutical composition and will essentially consist in:
(a) a botulinum toxin type A2,
(b) a surfactant, and
(c) water.

In the abovementioned pharmaceutical compositions, the surfactant will be such that it stabilises the botulinum toxin.

A solid pharmaceutical composition according to the invention can be obtained for example by lyophilising a sterile water solution containing the components (a) and (b) as mentioned previously. A liquid pharmaceutical composition according to the invention will be obtained by mixing the solid (e.g. lyophilised) mixture of components (a) and (b) with sterile water.

According to the invention, the concentrations of said components (a) and (b) in the solution to be lyophilised / the liquid pharmaceutical composition will preferably be as follows:
- the solution will contain from 50 to 3,000 LD₅₀ units of botulinum neurotoxin complex type A2 or high purity botulinum neurotoxin type A2 per ml of solution, more preferably from 100 to 2,500 LD₅₀ units of botulinum neurotoxin complex type A2 or high purity botulinum neurotoxin type A2 per ml of solution and most preferably from 100 to 2,000 LD₅₀ units of botulinum neurotoxin complex type A2 or high purity botulinum neurotoxin type A2 per ml of solution;
- the concentration of surfactant will be from above critical micellar concentration to a concentration of 1% v/v, and notably from about 0.005% to 0.02% v/v in the case of polysorbate 80.

Preferably, the surfactant will be a non-ionic surfactant. Non-ionic surfactants include notably polysorbates and block copolymers like poloxamers (i.e. copolymers of polyethylene and propylene glycol). According to a preferred variant of the invention, the surfactant will be a polysorbate. More preferably, a polysorbate included in a composition according to the instant invention will have a mean polymerisation degree of from 20 to 100 monomer units (preferably about 80), and may for example be polysorbate 80. Preferably also, the polysorbate should be vegetable-derived.

According to a preferred execution mode of the invention, the solid or liquid pharmaceutical composition will also contain a crystalline agent.

By crystalline agent is meant an agent which, *inter alia*, would maintain a mechanically strong cake structure to lyophilised botulinum neurotoxin complex or high purity botulinum neurotoxin. When included in solid formulations, crystalline agents also have a bulking effect. Crystalline agents notably include sodium chloride. Contrarily to what was taught in the prior art (see e.g. Goodnough, M.C. and Johnson, E.A., Applied and Environmental Microbiology (1992), 58(10), 3426-3428), the use of sodium chloride for this type of compositions further improves the stability of the botulinum toxin composition.

According to yet another preferred execution mode of the invention, the solid or liquid pharmaceutical composition will also contain a buffer to maintain pH from 5.5 to 7.5.

The buffer can be any buffer able to maintain the adequate pH. Preferably, the buffer for compositions according to the invention will be chosen from the group consisting of succinate and an amino acid like histidine. In particular, the buffer will be histidine. Preferably, the pH will be at least equal to 5.5 or 5.8, and most preferably at least equal to 6.0 or 6.5. Preferably also, the pH will be equal to or less than 7.5 or 7.0, more preferably equal to or less than 6.8.

Preferably, the solid or liquid pharmaceutical composition of the invention may also contain a disaccharide.

The disaccharide used in compositions according to the invention will preferably be chosen from the group consisting of sucrose, trehalose, mannitol and lactose. The disaccharide used in compositions according to the invention will more preferably be chosen from the group consisting of sucrose and trehalose. In particular, the disaccharide used in compositions according to the invention will be sucrose. Preferably, the disaccharide will be present in the pharmaceutical compositions of the instant invention, particularly when the compositions are in a solid form.

The instant invention therefore notably relates to a solid or liquid pharmaceutical composition comprising:
(a) botulinum neurotoxin complex type A2 or high purity botulinum neurotoxin type A2,
(b) a surfactant,
(c) a crystalline agent,
(d) a buffer to maintain pH between 5.5 to 7.5.

Preferably, a disaccharide will also be included in the pharmaceutical compositions according to the present invention, especially when they are in a solid form.

According to this variant of the invention, a solid pharmaceutical composition can be obtained by lyophilising a sterile water solution containing the components (a) to (d) as mentioned previously. A liquid pharmaceutical composition according to the invention will be obtained by mixing a solid (e.g. lyophilized) mixture of said components (a) to (d) with sterile water.

According to the invention, the concentrations of said components (a) to (d) in the solution to be lyophilised / the liquid pharmaceutical composition will preferably be as follows:
- the solution will contain from 50 to 3,000 LD₅₀ units of botulinum neurotoxin complex type A2 or high purity botulinum neurotoxin type A2 per ml of solution, more preferably from 100 to 2,500 LD₅₀ units of botulinum neurotoxin complex type A2 or high purity botulinum neurotoxin type A2 per ml of solution and most preferably from 100 to 2,000 LD₅₀ units of botulinum neurotoxin complex type A2 or high purity botulinum neurotoxin type A2 per ml of solution;
- the concentration of surfactant will be from above critical micellar concentration to a concentration of 1% v/v, and notably from about 0.005% to 0.02% v/v in the case of polysorbate 80;
- the concentration of crystalline agent will be from 0.1 to 0.5 M, more preferably from 0.1 to 0.4 M, notably about 0.15 to 0.3 M; and
- the concentration of buffer will be from 1 to 50 mM, more preferably from 5 to 20 mM, notably about 10 mM.

As mentioned earlier, the solid or liquid pharmaceutical formulation according to the invention may contain a disaccharide. In that case, the concentration of disaccharide in the solution to be lyophilised / the liquid pharmaceutical composition will be for example from 5 to 50 mM, preferably from 5 to 25 mM, more preferably from 10 to 20 mM, and notably about 11.7 mM.

According to a preferred execution mode of the invention, the mixture of the different components of the pharmaceutical composition (i.e. botulinum neurotoxin complex type A2 or high purity botulinum neurotoxin type A2, the surfactant and the optional excipients such as the crystalline agent, the buffer or the disaccharide) is lyophilised. The solid compositions thus obtained, which are also part of this invention, should preferably be stable for at least 12 months, more preferably for at least 18 months and in a more particularly preferred manner for at least 24 or even 36 months.

A composition according to the invention is considered stable during a certain period of time if at least 70% of the initial toxicity, as evaluated by assessing the LD₅₀ in mice or by any method validated with respect to the LD₅₀ mouse assay (i.e. a method allowing a conversion of its results into LD₅₀ units), is maintained over said period of time (cf. the part entitled "mouse toxicity assay" concerning the LD₅₀ mouse assay).

Pharmaceutical compositions according to the invention can be used for preparing medicaments intended to treat a disease / a condition / a syndrome chosen from the following:
❖ ophtalmological disorders selected from the group consisting of blepharospasm, strabismus (including restrictive or myogenic strabismus), amblyopia, oscillopsia, protective ptosis, therapeutic ptosis for corneal protection, nystagmus, estropia, diplopia, entropion, eyelid retraction, orbital myopathy, heterophoria, concomitant misalignment, nonconcomitant misalignment, primary or secondary esotropia or exotropia, internuclear ophthalmoplegia, skew deviation, Duane's syndrome and upper eyelid retraction;
❖ movement disorders including hemifacial spasm, torticollis, spasticity of the child or of the adult (e.g. in cerebral palsy, post-stroke, multiple sclerosis, traumatic brain injury or spinal cord injury patients), idiopathic focal dystonias, muscle stiffness, Writer's cramp, hand dystonia, VI nerve palsy, oromandibular dystonia, head tremor, tardive dyskinesia, tardive dystonia, occupational cramps (including musicians' cramp), facial nerve palsy, jaw closing spasm, facial spasm, synkinesia, tremor, primary writing tremor, myoclonus, stiff-person-syndrome, foot dystonia, facial paralysis, painful-arm-and-moving-fingers-syndrome, tic disorders, dystonic tics, Tourette's syndrome, neuromyotonia, trembling chin, lateral rectus palsy, dystonic foot inversion, jaw dystonia, Rabbit syndrome, cerebellar tremor, III nerve palsy, palatal myoclonus, akasthesia, muscle cramps, IV nerve palsy, freezing-of-gait, extensor truncal dystonia, post-facial nerve palsy synkinesis, secondary dystonia, Parkinson's disease, Huntington's chorea, epilepsy, off period dystonia, cephalic tetanus, myokymia and benign cramp-fasciculation syndrome;
❖ otorhinolaryngological disorders including spasmodic dysphonia, hypersalivation, sialorrhoea, otic disorders, hearing impairment, ear click, tinnitus, vertigo, Meniere's disease, cochlear nerve dysfunction, stuttering, cricopharyngeal dysphagia, bruxism, closure of larynx in chronic aspiration, vocal fold granuloma, ventricular dystonia, ventricular dysphonia, mutational dysphonia, trismus, snoring, voice tremor, aspiration, tongue protrusion dystonia, palatal tremor, deep bite of lip and laryngeal dystonia;
❖ gastrointestinal disorders including achalasia, anal fissure, constipation, temperomandibular joint dysfunction, sphincter of Oddi dysfunction, sustained sphincter of Oddi hypertension, intestinal muscle disorders, puborectalis syndrome, anismus, pyloric spasm, gall bladder dysfunction, gastrointestinal or oesophageal motility dysfunction, diffuse oesophageal spasm, oesophageal diverticulosis and gastroparesis;
❖ urogenital disorders including detrusor sphincter dyssynergia, detrusor hyperreflexia, neurogenic bladder dysfunction (e.g. in Parkinson's disease, spinal cord injury, stroke or multiple sclerosis patients), bladder spasms, urinary incontinence, urinary retention, hypertrophied bladder neck, voiding dysfunction, interstitial cystitis, vaginismus, endometriosis, pelvic pain, prostate gland enlargement (Benign Prostatic Hyperplasia), prostatodynia, prostate cancer and priapism;
❖ dermatological disorders including hyperhidrosis (including axillary hyperhidrosis, palmar hyperhidrosis and Frey's syndrome), bromhidrosis, cutaneous cell proliferative disorders (including psoriasis), skin wounds and acne;
❖ pain disorders including back pain (upper back pain, lower back pain), myofascial pain, tension headache, fibromyalgia, painful syndromes, myalgia, migraine, whiplash, joint pain, post-operative pain, pain not associated with a muscle spasm and pain associated with smooth muscle disorders;
❖ inflammatory disorders including pancreatitis, neurogenic inflammatory disorders (including gout, tendonitis, bursitis, dermatomyositis and ankylosing spondylitis);
❖ secretory disorders such as excessive gland secretions, mucus hypersecretion and hyperlacrimation, holocrine gland dysfunction;
❖ respiratory disorders including rhinitis (including allergic rhinitis), COPD, asthma and tuberculosis;
❖ hypertrophic disorders including muscle enlargement, masseteric hypertrophy, acromegaly and neurogenic tibialis anterior hypertrophy with myalgia;
❖ articular disorders including tennis elbow (or epicondilytis of the elbow), inflammation of joints, coxarthrosis, hip osteoarthritis, rotator muscle cap pathology of the shoulder, rheumatoid arthritis and carpal tunnel syndrome;
❖ endocrine disorders like type 2 diabetes, hyperglucagonism, hyperinsulinism, hypoinsulinism, hypercalcemia, hypocalcemia, thyroid disorders (including Grave's disease, thyroiditis, Hashimoto's thyroiditis, hyperthyroidism and hypothyroidism), parathyroid disorders (including hyperparathyroidism and hypoparathyroidism), Cushing's syndrome and obesity;
❖ autoimmune diseases like systemic lupus erythemotosus;
❖ proliferative diseases including paraganglioma tumors, prostate cancer and bone tumors;
❖ traumatic injuries including sports injuries, muscle injuries, tendon wounds and bone fractures; and
❖ veterinary disorders (e.g. immobilisation of mammals, equine colic, animal achalasia or animal muscle spasms)

Pharmaceutical compositions according to the invention can also be used for cosmetic treatments including cosmetic treatments of the following cosmetic disorders:
❖ skin defects;
❖ facial asymmetry;
❖ wrinkles including glabellar frown lines and facial wrinkles;
❖ downturned mouth;
❖ hair loss; and
❖ body odours.

Preferably, pharmaceutical compositions according to the invention will be used for preparing medicaments intended to treat a disease / a condition / a syndrome chosen from the following:
❖ ophtalmological disorders selected from the group consisting of blepharospasm, strabismus (including restrictive or myogenic strabismus), amblyopia, protective ptosis, therapeutic ptosis for corneal protection and upper eyelid retraction;
❖ movement disorders selected from the group consisting of hemifacial spasm, torticollis, cerebral palsy spasticity of the child, spasticity of the adult in post-stroke, multiple sclerosis, traumatic brain injury or spinal cord injury patients, idiopathic focal dystonias, muscle stiffness, Writer's cramp, hand dystonia, VI nerve palsy, oromandibular dystonia, head tremor, tardive dyskinesia, tardive dystonia, occupational cramps (including musicians' cramp), facial nerve palsy, jaw closing spasm, facial spasm, synkinesia, tremor, primary writing tremor, myoclonus, stiff-person-syndrome, foot dystonia, facial paralysis, painful-arm-and-moving-fingers-syndrome, tic disorders, dystonic tics, Tourette's syndrome, neuromyotonia, trembling chin, lateral rectus palsy, dystonic foot inversion, jaw dystonia, Rabbit syndrome, cerebellar tremor, III nerve palsy, palatal myoclonus, akasthesia, muscle cramps, IV nerve palsy, freezing-of-gait, extensor truncal dystonia, post-facial nerve palsy synkinesis, secondary dystonia, off period dystonia, cephalic tetanus, myokymia and benign cramp-fasciculation syndrome;
❖ otorhinolaryngological disorders selected from the group consisting of spasmodic dysphonia, hypersalivation, sialorrhoea, ear click, tinnitus, vertigo, Meniere's disease, cochlear nerve dysfunction, stuttering, cricopharyngeal dysphagia, bruxism, closure of larynx in chronic aspiration, vocal fold granuloma, ventricular dystonia, ventricular dysphonia, mutational dysphonia, trismus, snoring, voice tremor, aspiration, tongue protrusion dystonia, palatal tremor and laryngeal dystonia;
❖ gastrointestinal disorders selected from the group consisting of achalasia, anal fissure, constipation, temperomandibular joint dysfunction, sphincter of Oddi dysfunction, sustained sphincter of Oddi hypertension, intestinal muscle disorders, puborectalis syndrome, anismus, pyloric spasm, gall bladder dysfunction, gastrointestinal or oesophageal motility dysfunction, diffuse oesophageal spasm, oesophageal diverticulosis and gastroparesis;
❖ urogenital disorders selected from the group consisting of detrusor sphincter dyssynergia, detrusor hyperreflexia, neurogenic bladder dysfunction in Parkinson's disease, spinal cord injury, stroke or multiple sclerosis patients, bladder spasms, urinary incontinence, urinary retention, hypertrophied bladder neck, voiding dysfunction, interstitial cystitis, vaginismus, endometriosis, pelvic pain, prostate gland enlargement (Benign Prostatic Hyperplasia), prostatodynia, prostate cancer and priapism;
❖ dermatological disorders selected from the group consisting of axillary hyperhidrosis, palmar hyperhidrosis, Frey's syndrome, bromhidrosis, psoriasis, skin wounds and acne;
❖ pain disorders selected from the group consisting of upper back pain, lower back pain, myofascial pain, tension headache, fibromyalgia, myalgia, migraine, whiplash, joint pain, post-operative pain and pain associated with smooth muscle disorders;
❖ inflammatory disorders selected from the group consisting of pancreatitis, gout, tendonitis, bursitis, dermatomyositis and ankylosing spondylitis;
❖ secretory disorders selected from the group consisting of excessive gland secretions, mucus hypersecretion and hyperlacrimation and holocrine gland dysfunction;
❖ respiratory disorders selected from the group consisting of non-allergic rhinitis, allergic rhinitis, COPD and asthma;
❖ hypertrophic disorders selected from the group consisting of muscle enlargement, masseteric hypertrophy, acromegaly and neurogenic tibialis anterior hypertrophy with myalgia;
❖ articular disorders selected from the group consisting of tennis elbow (or epicondilytis of the elbow), inflammation of joints, coxarthrosis, hip osteoarthritis, rotator muscle cap pathology of the shoulder, rheumatoid arthritis and carpal tunnel syndrome;
❖ endocrine disorders selected from the group consisting of type 2 diabetes, hypercalcemia, hypocalcemia, thyroid disorders, Cushing's syndrome and obesity;
❖ prostate cancer; and
❖ traumatic injuries selected from the group consisting of sports injuries, muscle injuries, tendon wounds and bone fractures;
or for performing cosmetic treatments wherein the cosmetic disorder to be treated is selected from the group consisting of:
❖ skin defects;
❖ facial asymmetry;
❖ wrinkles selected from glabellar frown lines and facial wrinkles;
❖ downturned mouth; and
❖ hair loss.

More preferably, pharmaceutical compositions according to the invention will be used for preparing medicaments intended to treat a disease / a condition / a syndrome chosen from the following:
❖ ophtalmological disorders selected from the group consisting of blepharospasm and strabismus;
❖ movement disorders selected from the group consisting of hemifacial spasm, torticollis, cerebral palsy spasticity of the child and arm or leg spasticity of the adult in post-stroke, multiple sclerosis, traumatic brain injury or spinal cord injury patients;
❖ otorhinolaryngological disorders selected from the group consisting of spasmodic dysphonia, hypersalivation, sialorrhoea, cricopharyngeal dysphagia, bruxism, closure of larynx in chronic aspiration, ventricular dystonia, ventricular dysphonia, mutational dysphonia, trismus, snoring, voice tremor, tongue protrusion dystonia, palatal tremor and laryngeal dystonia;
❖ gastrointestinal disorders selected from the group consisting of achalasia, anal fissure, constipation, temperomandibular joint dysfunction, sphincter of Oddi dysfunction, sustained sphincter of Oddi hypertension, intestinal muscle disorders, anismus, pyloric spasm, gall bladder dysfunction, gastrointestinal or oesophageal motility dysfunction and gastroparesis;
❖ urogenital disorders selected from the group consisting of detrusor sphincter dyssynergia, detrusor hyperreflexia, neurogenic bladder dysfunction in Parkinson's disease, spinal cord injury, stroke or multiple sclerosis patients, bladder spasms, urinary incontinence, urinary retention, hypertrophied bladder neck, voiding dysfunction, interstitial cystitis, vaginismus, endometriosis, pelvic pain, prostate gland enlargement (Benign Prostatic Hyperplasia), prostatodynia, prostate cancer and priapism;
❖ dermatological disorders selected from the group consisting of axillary hyperhidrosis, palmar hyperhidrosis, Frey's syndrome, bromhidrosis, psoriasis, skin wounds and acne;
❖ pain disorders selected from the group consisting of upper back pain, lower back pain, myofascial pain, tension headache, fibromyalgia, myalgia, migraine, whiplash, joint pain, post-operative pain and pain associated with smooth muscle disorders;
❖ inflammatory disorders selected from the group consisting of pancreatitis and gout;
❖ hyperlacrimation;
❖ respiratory disorders selected from the group consisting of non-allergic rhinitis, allergic rhinitis, COPD and asthma;
❖ masseteric hypertrophy;
❖ articular disorders selected from the group consisting of tennis elbow (or epicondilytis of the elbow), inflammation of joints, coxarthrosis, hip osteoarthritis, rotator muscle cap pathology of the shoulder, rheumatoid arthritis and carpal tunnel syndrome;
❖ obesity;
❖ traumatic injuries selected from the group consisting of muscle injuries, tendon wounds and bone fractures;
or for performing cosmetic treatments wherein the cosmetic disorder to be treated is selected from the group consisting of:
❖ skin defects;
❖ facial asymmetry;
❖ wrinkles selected from glabellar frown lines and facial wrinkles;
❖ downturned mouth; and
❖ hair loss.

In a particularly preferred manner, pharmaceutical compositions according to the invention will be used for preparing medicaments intended to treat a disease / a condition / a syndrome chosen from the following: blepharospasm, hemifacial spasm, torticollis, cerebral palsy spasticity of the child and arm or leg spasticity of the adult in post-stroke, multiple sclerosis, traumatic brain injury or spinal cord injury patients, axillary hyperhidrosis, palmar hyperhidrosis, Frey's syndrome, skin wounds, acne, upper back pain, lower back pain, myofascial pain, migraine, tension headache, joint pain, tennis elbow (or epicondilytis of the elbow), inflammation of joints, coxarthrosis, hip osteoarthritis, rotator muscle cap pathology of the shoulder, muscle injuries, tendon wounds and bone fractures;
or for performing cosmetic treatments wherein the cosmetic disorder to be treated is selected from the group consisting of:
❖ skin defects;
❖ facial asymmetry; and
❖ wrinkles selected from glabellar frown lines and facial wrinkles.

The dose of botulinum neurotoxin complex type A2 or high purity botulinum neurotoxin type A2 which shall be needed for the treatment of the diseases / disorders mentioned above varies depending on the disease / disorder to be treated, administration mode, age and body weight of the patient to be treated and health state of the latter, and it is the treating physician or veterinarian that will eventually make the decision. Such a quantity determined by the treating physician or veterinarian is called here "therapeutically effective dose".

Moreover, the invention relates to the use of botulinum toxin type A2 for the preparation of a medicament intended to treat the diseases / conditions / syndromes mentioned previously.

It also relates to a method of treating cosmetic disorders selected from the group consisting of:
❖ skin defects;
❖ facial asymmetry;
❖ wrinkles selected from glabellar frown lines and facial wrinkles;
❖ downturned mouth; and
❖ hair loss;
said method comprising the administration of botulinum toxin type A2 to the area affected by the cosmetic disorder.

For botulinum neurotoxin complex or high purity botulinum neurotoxin, this therapeutically effective dose is often expressed as a function of the corresponding LD₅₀. By LD₅₀ should be understood in the present application the median intraperitoneal dose in mice injected with botulinum neurotoxin complex or high purity botulinum neurotoxin that causes death of half of said mice within 96 hours.

The Applicant has now surprisingly found that botulinum toxin type A2 not only has a biological activity similar to that of the other botulinum neurotoxins, but also can have the major advantage of a much longer duration of action than any other known botulinum toxin (as shown for example by the rat muscle force assay described in the "Pharmacological study, Part I"), making it preferred over botulinum neurotoxins of other serotypes for any therapeutic use known for botulinum toxin type A1.

The Applicant has now surprisingly found that botulinum toxin type A2 not only has a biological activity similar to that of the other botulinum neurotoxins, but also can have the major advantage of a much faster rate of onset of muscular paralysis than any other known botulinum toxin (as shown for example by the rat muscle force assay described in the "Pharmacological study, Part II"), making it preferred over botulinum neurotoxins of other serotypes for any therapeutic use known for botulinum toxin type A1.

The Applicant has now surprisingly found that botulinum toxin type A2 not only has a biological activity similar to that of the other botulinum neurotoxins, but also can have the major advantage of a significantly greater intramuscular safety margin than any other known botulinum toxin (as shown for example by the intramuscular safety margin assay described in the "Pharmacological study, Part III"), making it preferred over botulinum neurotoxins of other serotypes for any therapeutic use known for botulinum toxin type A1.

The Applicant has now surprisingly found that botulinum toxin type A2 not only has a biological activity similar to that of the other botulinum neurotoxins, but also can have the major advantage of a selective action on inhibition of skeletal muscle contraction compared to other known botulinum toxins (as shown for example by the "Criteria for determination of selectivity for skeletal muscles" described in the "Pharmacological study, Part IV"). Since botulinum toxin type A2 can have less side-effects with respect to neighbouring smooth muscles, it may be preferred over botulinum neurotoxins of other serotypes for any skeletal muscle-related therapeutic use known for botulinum toxin type A1.

The Applicant has now surprisingly found that botulinum toxin type A2 not only has a biological activity similar to that of the other botulinum neurotoxins, but also can have the major advantage of a selective action on inhibition of pain-related (i.e. nociceptive) nerve cell function compared to other known botulinum toxins (as shown for example by the "Criteria for determination of selectivity for nociceptive neurotransmission" described in the "Pharmacological study, Part V"). Since botulinum toxin type A2 can have less side-effects with respect to neighbouring striated muscles, it may be preferred over botulinum neurotoxins of other serotypes for any pain-related therapeutic use known for botulinum toxin type A1.

The term "about" refers to an interval around the considered value. As used in this patent application, "about X" means an interval from X minus 10% of X to X plus 10% of X, and preferably an interval from X minus 5% of X to X plus 5% of X.

Unless they are defined differently, all the technical and scientific terms used here have the same meaning as that usually understood by an ordinary specialist in the field to which this invention belongs. Similarly, all publications, patent applications, all patents and all other references mentioned here are incorporated by way of reference (where legally permissable).

The term "comprising" or "having" as used herein is to be interpreted as meaning both "including" and "consisting of".

The following examples are presented to illustrate the above and must in no case be considered as a limit to the scope of the invention.

### EXAMPLES

### Example 1: (NOT ACCORDING TO THE INVENTION)

A liquid pharmaceutical composition containing the following components is prepared:

| *Clostridium botulinum* type A1 neurotoxin complex | 2,000 LD₅₀ units/ml |
|---|---|
| Sucrose | 11.7 mM |
| Histidine | 10 mM |
| Sodium chloride | 0.3 M |
| Polysorbate 80 | 0.01% v/v |
| pH | 6.5 |

The mixture containing nominally 2,000 LD₅₀ units of botulinum toxin per ml is lyophilised in a sterilised vial which is then sealed. The solid composition obtained is stable for at least 12 months when stored at a temperature between 2 and 8 °C and at least 6 months at 23 to 27 °C.

### Example 2: (NOT ACCORDING TO THE INVENTION)

A liquid pharmaceutical composition containing the following components is prepared:

| *Clostridium botulinum* type A1 neurotoxin complex | 500 LD₅₀ units/ml |
|---|---|
| Sucrose | 11.7 mM |
| Histidine | 10 mM |
| Sodium chloride | 0.3 M |
| Polysorbate 80 | 0.01% v/v |
| pH | 6.5 |

The liquid composition thus prepared is sealed in a syringe type device with no liquid/gaseous interface. Stored in these conditions, it is stable for at least one month at 23 to 27 °C and at least six months at 2-8 °C.

### Example 3: (NOT ACCORDING TO THE INVENTION)

A liquid pharmaceutical composition containing the following components is prepared:

| *Clostridium botulinum* type A1 neurotoxin complex | 500 LD₅₀ units/ml |
|---|---|
| Sucrose | 11.7 mM |
| Histidine | 10 mM |
| Sodium chloride | 0.15 M |
| Polysorbate 80 | 0.01% v/v |
| pH | 6.5 |

The liquid composition composition thus prepared is sealed in a syringe type device with no liquid/gaseous interface. Stored in these conditions, it is stable for at least one month at 23 to 27 °C and at least six months at 2-8 °C

### Example 4: (ACCORDING TO THE INVENTION)

A liquid pharmaceutical composition containing the following components is prepared:

| *Clostridium botulinum* type A2 neurotoxin complex | 500 LD₅₀ units/ml |
|---|---|
| Sucrose | 11.7 mM |
| Histidine | 10 mM |
| Sodium chloride | 0.15 M |
| Polysorbate 80 | 0.01% v/v |
| pH | 6.5 |

The liquid composition composition thus prepared is sealed in a syringe type device with no liquid/gaseous interface.

### Example 5:

A patient in his fifties suffers from cervical dystonia. He receives by intramuscular injection the liquid pharmaceutical composition of Example 4 (1 ml; 500 LD₅₀ units) is injected, the total dose being divided into the most active muscles of his neck. Relief of his symptoms is observed for more than 20 weeks.

### ANALYTICAL METHODS

### Mouse toxicity assay

A mouse toxicity assay can be used to measure the toxicity of botulinum neurotoxin complex or high purity botulinum neurotoxin. In the assay, a standard diluent will be used to prepare a range of dilutions at or about the estimated LD₅₀ value. The range and scale of dilutions is arranged so as to establish an accurate LD₅₀ value.

Mice are injected intraperitoneally with a known and standardised volume of diluted toxin. After 96 hours, the number of deaths and survivors in each dilution group will be recorded. The LD₅₀ value is the median dose which kills half of the injected animals within 96 hours.

A composition according to the invention is considered stable over a certain period of time if at least 70% of the initial toxicity is maintained over said period of time relative to a reference preparation.

### PHARMACOLOGICAL STUDY PART I

### Rat muscle force assay

The rat muscle force assay is a method capable of determining duration of paralysis by periodic measurement of force exerted by the tricep surae group of muscles (Gastrocnemius, Plantaris and Soleus) in the hind limbs of a rat before and after administration of botulinum toxin.

Adult, male Sprague-Dawley rats are randomly assigned to groups containing 8 animals each. After adequate anesthesia, the hindquarters and back legs of the animals are shaved. The gastrocnemius muscle of the left leg is injected with formulated botulinum toxin in 0.1 ml gelatine phosphate buffer. Groups receive equimolar amounts of either botulinum toxin type A2 or botulinum toxin type A1. Control animals receive an injection of gelatine phosphate buffer (0.1 ml) each. After adequate anesthesia, the hindquarters and back legs of the animals are shaved. The gastrocnemius muscle of the left leg is injected with a single dose of Dysport in gelatine phosphate buffer reconstituted to give either 1.0 U / 0.1 ml or 0.1 U / 0.1 ml). Control animals receive an injection of gelatine phosphate buffer (0.1 ml).

Muscle force of the tricipes surae group (Gastrocnemius, Plantaris, and Soleus) measured before injection and after injection at 12, 24 and 72 hours. Additional measurements are made at periodically over a course of several weeks. Body weights are recorded at the same time intervals.

To measure force development, animals are placed in a prone position in an apparatus that allows the animal to be secured in a reproducible position with limited mobility of the lower leg except at the tibiotarsal join. A force/displacement ergometer is calibrated and secured to the forefoot between the first and second footpads by a lightweight chain such that the tibiotarsal angle is 90 degrees. The voltage signal from the force transducer is processed via a computerized data acquisition system.

A stainless steel stimulating electrode (cathode) is placed transcutaneously near the sciatic nerve midway between the posterior ischeal spine and the greater femoral trochanter. Another stainless steel stimulating electrode (anode) is inserted 3 mm subdermally in the midline of the lower back.

Electrode sites are tattooed to ensure reproducible electrode placement at all time points. The sciatic nerve is stimulated with 0.5 pulses per second and a stimulus time of 0.5 ms. The stimulation voltage is determined by increasing the voltge until force reached a maximum and increasing the voltage an additional 10 %.

Measurment of muscle force using this method shows that recovery of muscles from paralysis by type A2 botulinum toxin occurs over a significantly prolonged duration of time when compared to recovery of muscles from paralysis by type A1 botulinum toxin.

### PHARMACOLOGICAL STUDY PART II

### Rat muscle force assay

The rat muscle force assay is a method capable of determining duration of paralysis by periodic measurement of force exerted by the tricep surae group of muscles (Gastrocnemius, Plantaris and Soleus) in the hind limbs of a rat before and after administration of botulinum toxin.

Adult, male Sprague-Dawley rats are randomly assigned to groups containing 8 animals each. After adequate anesthesia, the hindquarters and back legs of the animals are shaved. The gastrocnemius muscle of the left leg is injected with formulated botulinum toxin in 0.1 ml gelatine phosphate buffer. Groups receive equimolar amounts of either botulinum toxin type A2 or botulinum toxin type A1. Control animals receive an injection of gelatine phosphate buffer (0.1 ml) each.

After adequate anesthesia, the hindquarters and back legs of the animals are shaved. The gastrocnemius muscle of the left leg is injected with a single dose of Dysport in gelatine phosphate buffer reconstituted to give either 1.0 U / 0.1 ml or 0.1 U / 0.1 ml). Control animals receive an injection of gelatine phosphate buffer (0.1 ml).

Muscle force of the tricipes surae group (Gastrocnemius, Plantaris, and Soleus) measured before injection and after injection at 12, 24 and 72 hours. Additional measurements are made at periodically over a course of several weeks. Body weights are recorded at the same time intervals.

To measure force development, animals are placed in a prone position in an apparatus that allows the animal to be secured in a reproducible position with limited mobility of the lower leg except at the tibiotarsal join. A force/displacement ergometer is calibrated and secured to the forefoot between the first and second footpads by a lightweight chain such that the tibiotarsal angle is 90 degrees. The voltage signal from the force transducer is processed via a computerized data acquisition system.

A stainless steel stimulating electrode (cathode) is placed transcutaneously near the sciatic nerve midway between the posterior ischeal spine and the greater femoral trochanter. Another stainless steel stimulating electrode (anode) is inserted 3 mm subdermally in the midline of the lower back.

Electrode sites are tattooed to ensure reproducible electrode placement at all time points. The sciatic nerve is stimulated with 0.5 pulses per second and a stimulus time of 0.5 ms. The stimulation voltage is determined by increasing the voltge until force reached a maximum and increasing the voltage an additional 10 %.

Measurement of muscle force using this method shows that rate of onset of paralysis induced by botulinum type A2 toxin is significantly faster when compared to onset of paralysis of muscles induced by type A1 toxin.

### PHARMACOLOGICAL STUDY PART III

### Intramuscular safety margin assay

For determination of intramuscular LD₅₀, CD1 mice are randomly assigned to groups containing 8 animals each. The gastrocnemius muscle of the left leg is injected with formulated botulinum toxin in 0.1 ml gelatine phosphate buffer. Groups receive equimolar amounts of either botulinum type A2 toxin or type A1 toxin, each group receiving one of a range of doses. The i.m. LD₅₀ is calculated (Spearmann-Karber analysis) as the dose at which 50% of the mice died following i.m. injection.

For determination of half maximal muscle weakness (ED₅₀), the Digit Abduction Scoring assay (DAS) assay is used (Aoki KR, Toxicon (2001), 39, 1815-1820). CD1 mice are assigned randomly into groups of 10 each. The gastrocnemius muscle of the left leg is injected with formulated botulinum toxin in 0.1 ml gelatine phosphate buffer. Groups receive equimolar amounts of either botulinum type A2 toxin or botulinum type A1 toxin, each group receiving one of a range of doses. After a fixed period following injection, mice are briefly suspended by the tail to generate digit abduction as part of the characteristic startle response in this position. The abduction of the digits of the limb injected is scored on a scale of 0 to 4, where 0 is normal abduction and 4 is the maximal reduction in abduction of the digits and extension of the limb. ED₅₀ was calculated as the dose resulting in a digit abduction score of 2.

Measurement of intramuscular LD₅₀ to intramuscular ED₅₀ ratio from results obtained using these two methods shows that intramuscular safety margin of muscle weaking produced by botulinum type A2 toxin is greater when compared to onset of paralysis of muscles induced by botulinum type A1 toxin.

### PHARMACOLOGICAL STUDY PART IV

### A) Intercostal muscle assay:

For determination of inhibition of skeletal muscle neuromuscular junctions (NMJs), the intercostal muscle assay is used (as described in UK Patent application No. GB 2 398 636). Wistar rats weighing approximately 275 g are killed by cervical dislocation. The rib cage is dissected from each animal, and separated into multiple sections by careful dissection along the spinal column. For each preparation (consisting of two ribs and attached muscle) one intercostal nerve is carefully dissected to reveal approximately 1 - 2 mm of nerve bundle. The preparation is revived for approximately 15-20 minutes before being returned to a Petri dish containing 10 ml of oxygenated Lillies Ringers buffer. The dissected intercostal nerve is connected via a suction electrode to a stimulator (Grass Instruments Model S48), with a return electrode placed in the media. The tissue preparation is connected to an amplifier and force transducer (Grass Instruments Model P122 and FT03, respectively), so as to allow measurement of muscle force generated.

The preparation is stimulated at a supramaximal voltage, and contractile responses recorded. After a stabilization period, replicate organ baths are then exposed to a known molar amount of the botulinum toxin to be tested, and magnitude of twitch recorded. Control preparations are treated with diluent only.

### B) Guinea pig ileum assay:

For determination of inhibition of smooth muscle contraction, the guinea pig ileum assay is used (a modification of the method described by Mackenzie IJ et al, Neuroscience 7, 1982, 997-1006). Male Hartley guinea pigs (Charles River, France) weighing between 300-450 g, are killed by cervical dislocation. The distal part of the ileum is removed and segments 1.5-2 cm long are mounted on tissue fitted holders, between two parallel platinum wire electrodes. This assembly is placed in a 20 ml organ bath containing modified Krebs solution under a tension of 1g at 37 °C and gassed with 95% O₂ / 5% CO₂. Contractile responses are measured using force displacement transducers (Statham UC₂) coupled to a Gould RS3400 polygraph.

After 1 h equilibration period, the tissues are stimulated electrically between 0.05 Hz and 0.2 Hz with square wave pulses of 0.5 ms to 1 ms duration and supramaximal voltage is then determined. After a stabilization period, replicate organ baths are then exposed to a known molar amount of the botulinum toxin to be tested, and magnitude of twitch recorded. Control preparations are treated with diluent only. Additional replicate organ baths are treated with 0.25 µM tetrodotoxin or 0.56 µM atropine to confirm that contractions observed are due to release of acetylcholine from enteric neurons.

### C) Criteria for determination of selectivity for skeletal muscles:

The selectivity ratio for a certain botulinum toxin is defined as the value obtained at the test described in A) above divided by the value obtained at the test described in B) above, while the same molar quantity of active botulinum toxin to be tested is used in both tests.

The selectivity ratio thus found for botulinum type A2 toxin is found significantly superior to that of botulinum type A1 toxin.

### PHARMACOLOGICAL STUDY PART V

### A) Nociceptive nerve cell function assay:

For determination of inhibition of nociceptive nerve cell function, the embryonic dorsal root ganglion assay is used (as described by Welch MJ et al., Toxicon (2000), 38, 245-258). Dissociated nerve cells are prepared from dorsal root ganglia harvested from 15-day old foetal Sprague-Dawley rats, and plated out in Matrigel coated 24 well plates. One day after plating, the cells are treated with cytosine β-D-arabinofuranoside for 48 hours at a concentration of 10 micromolar. Cells are then maintained in tissue culture medium for 2 weeks under standard tissue culture conditions. Replicate cell cultures are then exposed to a known molar amount of the botulinum toxin to be tested. Control cells are treated with diluent only.

Following incubation with the botulinum toxin to be tested, substance P release was stimulated using a high potassium buffer, and measured by use of a substance P Enzyme Immunoassay (EIA) kit available commercially.

### B) Intercostal muscle assay:

For determination of inhibition of skeletal muscle neuromuscular junctions (NMJs), the intercostal muscle assay is used (as described in UK Patent application No. GB 2 398 636). Wistar rats weighing approximately 275 g are killed by cervical dislocation. The rib cage is dissected from each animal, and separated into multiple sections by careful dissection along the spinal column. For each preparation (consisting of two ribs and attached muscle) one intercostal nerve is carefully dissected to reveal approximately 1 - 2 mm of nerve bundle. The preparation is revived for approximately 15-20 minutes before being returned to a Petri dish containing 10 ml of oxygenated Lillies Ringers buffer. The dissected intercostal nerve is connected via a suction electrode to a stimulator (Grass Instruments Model S48), with a return electrode placed in the media. The tissue preparation is connected to an amplifier and force transducer (Grass Instruments Model P122 and FT03, respectively), so as to allow measurement of muscle force generated.

The preparation is stimulated at a supramaximal voltage, and contractile responses recorded. After a stabilization period, replicate organ baths are then exposed to a known molar amount of the botulinum toxin to be tested, and magnitude of twitch recorded. Control preparations are treated with diluent only.

### C) Criteria for determination of selectivity for nociceptive neurotransmission:

The selectivity ratio for a certain botulinum toxin is defined as the value obtained at the test described in A) above divided by the value obtained at the test described in B) above, while the same molar quantity of active botulinum toxin to be tested is used in both tests.

The selectivity ratio thus found for botulinum type A2 toxin is found significantly superior to that of botulinum type A1 toxin.

## Claims

1. A solid or liquid pharmaceutical composition comprising:
(a) botulinum neurotoxin complex type A2 or high purity botulinum neurotoxin type A2, and
(b) a surfactant,

2. A solid or liquid pharmaceutical composition according to claim 1 further comprising a crystalline agent.

3. A solid or liquid pharmaceutical composition according to claim 2 wherein the crystalline agent is sodium chloride.

4. A solid or liquid pharmaceutical composition according to one of claims 1 to 3 further comprising a buffer to maintain pH between 5.5 to 7.5.

5. A solid or liquid pharmaceutical composition according claim 4 wherein the buffer is maintaining a pH from 5.8 to 7.0.

6. A solid or liquid pharmaceutical composition according to any of claims 1 to 5 further comprising a disaccharide.

7. A solid or liquid pharmaceutical composition according to claim 6 wherein the disaccharide is chosen from the group consisting of sucrose, trehalose, lactose and mannitol.

8. A pharmaceutical composition according to any of claims 1 to 7 in which the surfactant is polysorbate 80.

9. As a medicament, botulinum toxin type A2.

10. Pharmaceutical composition comprising, as active principle, botulinum toxin type A2.

11. Use of botulinum toxin type A2 or a pharmaceutical composition according to any of claims 1 to 8 for the manufacture of a medicament intended to treat a disease / a condition / a syndrome chosen from the following: ophtalmological disorders, movement disorders, otorhinolaryngological disorders, gastrointestinal disorders, urogenital disorders, dermatological disorders, pain disorders, inflammatory disorders, secretory disorders, respiratory disorders, hypertrophic disorders, articular disorders, endocrine disorders, autoimmune diseases, proliferative diseases, traumatic injuries and veterinary disorders.

12. Use of botulinum toxin type A2 or a pharmaceutical composition according to any of claims 1 to 8 for treating cosmetic disorders selected from the group consisting of:
❖ skin defects;
❖ facial asymmetry;
❖ wrinkles selected from glabellar frown lines and facial wrinkles;
❖ downturned mouth; and
❖ hair loss;
said method comprising the administration of an effective dose botulinum toxin type A2 to the area affected by the cosmetic disorder.

13. A solid or liquid pharmaceutical composition substantially as herein described.

14. A composition, medicament or use, according to any preceding claim, having one or more or all of the characteristics selected from the group consisting of;
(a) a much longer duration of action;
(b) a much faster rate of onset of muscular paralysis;
(c) a significantly greater intramuscular safety margin;
(d) a selective action on inhibition of skeletal muscle contraction;
(e) a selective action on inhibition of pain-related (nociceptive) nerve cell function:
Wherein each characteristic is determined comparative to the same characteristic effect of botulinum toxin type A1.
